# EUROPEAN PATENT SPECIFICATION

(11) **EP 4 482 481 B1**
(45) Date of publication and mention of the grant of the patent: **12.11.2025**
(21) Application number: 23705290.7
(22) Date of filing: 20.02.2023
(51) Int. Cl.: A61K 31/18, A61K 31/35, A61K 31/4184, A61K 31/4402, A61K 31/4439, A61K 31/4704, A61K 31/4725, A61K 31/54, A61K 31/621, A61P 21/00, A61K 31/37, A61K 31/47, A61K 31/4709, A61K 31/5377, A61K 31/549

(54) **COMPOUNDS FOR USE IN THE TREATMENT OF DYSTROPHINOPATHIES**
VERBINDUNGEN ZUR VERWENDUNG BEI DER BEHANDLUNG VON DYSTROPHINOPATHIEN
COMPOSÉS À UTILISER DANS LE TRAITEMENT DES DYSTROPHINOPATHIES

(30) Priority: 21.02.2022 EP 22382147
(43) Date of publication of application: 01.01.2025
(73) Proprietor: Som Innovation Biotech, S.A., 08028 Barcelona (ES); Regents of the University of Minnesota, Minneapolis, MN 55455 (US)
(72) Inventor: PERICOT MOHR, Gal.la, 08028 Barcelona (ES); INSA BORONAT, Raúl, 08028 Barcelona (ES); HUERTAS GAMBÍN, Óscar, 08028 Barcelona (ES); REIG BOLAÑO, Núria, 08028 Barcelona (ES); ASAKURA, Atsushi, Saint Anthony, Minnesota 55418 (US); ASAKURA, Yoko, Saint Anthony, Minnesota 55418 (US); MONTOLIO DEL OLMO, Marisol, E-28032 Madrid (ES)
(74) Representative: ABG Intellectual Property Law, S.L.
(86) International application number: PCT/EP2023/054140
(87) International publication number: WO 2023/156645

(56) References cited:
- WO-A1-2021/025899
- SHIEH PERRY B ED - VINK ROBERT ET AL: "Emerging Strategies in the Treatment of Duchenne Muscular Dystrophy", NEUROTHERAPEUTICS, SPRINGER INTERNATIONAL PUBLISHING, CHAM, vol. 15, no. 4, 9 November 2018 (2018-11-09), pages 840 - 848, XP036651650, ISSN: 1933-7213, [retrieved on 20181109], DOI: 10.1007/S13311-018-00687-Z
- VERHAART INGRID E ET AL: "Therapeutic developments for Duchenne muscular dystrophy", NATURE REVIEWS NEUROLOGY, NATURE PUBLISHING GROUP UK, LONDON, vol. 15, no. 7, 30 May 2019 (2019-05-30), pages 373 - 386, XP036823394, ISSN: 1759-4758, [retrieved on 20190530], DOI: 10.1038/S41582-019-0203-3

## Description

### FIELD OF THE INVENTION

The present invention relates to compounds for use in the treatment and/or prevention of dystrophinopathies, and in particular of Duchenne muscular dystrophy.

### BAKGROUND OF THE INVENTION

Dystrophinopathies are X-linked progressive hereditary degenerative diseases of muscles caused by an absence or deficiency of dystrophin, a sarcolemmal protein. The dystrophinopathies cover a spectrum of disease severity. The mild end of the spectrum includes muscle cramps with myoglobinuria; whereas the severe end of the spectrum includes progressive muscle diseases such as Duchenne or Becker muscular dystrophy when skeletal muscle is primarily affected, or dilated cardiomyopathy when it is the heart that is primarily affected (Astejada et al., Reference Module in Biomedical Sciences, International Encyclopedia of Public Health, 2nd edition, 2017, Volume 5, 183-192; Darras et al., 2000 Sep 5 [Updated 2018 Apr 26]. In: Adam MP, Ardinger HH, Pagon RA, et al., editors. GeneReviews, [Internet]. Seattle (WA): University of Washington, Seattle; 1993-2021, PMID: 20301298).

Duchenne muscular dystrophy (DMD) affects one in 5,000 newborn males, and arises when the gene encoding the dystrophin protein is mutated. It is a progressive neuromuscular disease with clinical symptoms manifesting at 2-3 years of age, loss of ambulation in early teen years, and death by either respiratory insufficiency or cardiac failure in the following decade.

Becker muscular dystrophy (BMD) is characterized by similar symptoms to DMD, however with a later-onset skeletal muscle weakness, with some patients remaining ambulatory until their 30s. Nevertheless, heart failure still remains the most common cause of death in BMD patients. Mean age of death is in the mid-40s.

DMD-associated dilated cardiomyopathy (DCM) is characterized by left ventricular dilation and congestive heart failure, despite there usually being no clinical evidence of skeletal muscle disease. Females heterozygous for a DMD pathogenic variant are at increased risk for DCM. Heart failure in males typically presents itself between ages 20 and 40 and at a later stage in females.

Dystrophin, the protein affected in these diseases, provides structural integrity to the skeletal and cardiac muscle by linking the sarcolemmal actin filaments to the basement membrane protein complex, via the dystrophin associated protein complex (DAPC), to effect mechanotransduction. In dystrophinopathies, the entire DAPC is lost from the sarcolemma, causing aberrant mechanotransduction in muscle fibers.

Although the molecular mechanisms underlying dystrophinopathies have been extensively investigated, there is still no complete curative treatment available (Soblechero-Martin et al., Neuropathol Appl Neurobiol, 2021;00:1-13). The current standard of care includes corticoids, such as prednisone or deflazacort, to delay disease progression. Therapies based on dystrophin replacement at the protein or gene level are challenging due to the gene's large size, the wide distribution of the skeletal muscle throughout the body, and the possibility of immune response activation.

The upregulation of the utrophin protein has recently been gaining strength as one of the most promising alternative therapeutic strategies against dystrophinopathies. Utrophin, a dystrophin-related protein, is an autosomal homolog of dystrophin that can also bind to proteins of the DAPC and functionally replace dystrophin. Increasing the levels of utrophin results in partially restoring affected muscle functions in the dystrophin-deficient muscle fibers in *mdx* mice via increased levels of sarcolemmal bound utrophin. Furthermore, utrophin overexpression does not induce any immune response in dystrophinopathic patients due to its endogenous expression.

Recent studies based on high-throughput screening such as in myoblast cells have allowed identifying small molecules capable of inducing utrophin upregulation and thus rescuing muscle function, a number of which are currently undergoing clinical trials (Soblechero-Martin et al., Neuropathol Appl Neurobiol, 2021;00:1-13; Verhaart et al., Nature Reviews Neurology 15, 373-386 (2019)).

It is the aim of the present invention to identify further compounds capable of inducing utrophin upregulation to thus enrich the arsenal of therapies against dystrophinopathies.

### SUMMARY OF THE INVENTION

The inventors have surprisingly found a series of compounds capable of inducing utrophin upregulation, and which can therefore be useful to prevent and treat dystrophinopathic disease.

Thus, in a first aspect, the present invention relates to a compound selected from the group consisting of Difenpiramide, Veliflapon, Amopyroquine, Cloricromen, Alpelisib, Benorilate, Xipamide, Fabomotizole and Altizide, or a pharmaceutically acceptable salt thereof, for use in the treatment and/or prevention of a dystrophinopathy.

In a second aspect, the present invention relates to a combination comprising two or more agents selected from the group consisting of Difenpiramide, Veliflapon, Amopyroquine, Cloricromen, Alpelisib, Benorilate, Xipamide, Fabomotizole, Altizide, or a pharmaceutically acceptable salt thereof; ataluren, deflazacort, givinostat, pizuglanstat, ifetroban, vamorolone, idebenone, prednisone, prednisolone, 20-hydroxyecdysone, metformin and L-citrulline combination, MA-0211, tamoxifen, dextro epicatechin, exaluren, ARM-210, 2,6-disubstituted-pyiridazin-3(2H)-ones, N-Benzyl-p-toluenesulphonamide, MP-101, MT-002, rimeporide, tetracosactide hexaacetate, elamipretide, KER-050, SRP-5051, TXA-127, PB-1023, pamrevlumab, eteplirsen, golodirsen, vitolarsen, casimersen, ATL-1102, NS-089, renadirsen, WVEN-531, fordadistrogene movaparvovec, GNT-004, AAV1-follistatin viral vector, delandistrogene moxeparvovec, AT-702, AAVrh74-GALGT2, SGT-001, CAP-2003, CAP-1002; wherein at least one of said agents is selected from the group consisting of Difenpiramide, Veliflapon, Amopyroquine, Cloricromen, Alpelisib, Benorilate, Xipamide, Fabomotizole and Altizide, or a pharmaceutically acceptable salt thereof.

In a third aspect, the invention relates to a combination according to the second aspect of the invention, for use in the treatment and/or prevention of a dystrophinopathy.

### DESCRIPTION OF THE FIGURES

Figure 1 shows maximal twitch contractile force in response to electrical stimulation upon treatment with compounds of the invention.
Figure 2 shows tetanic contractile force in response to electrical stimulation upon treatment with compounds of the invention.
Figure 3 shows tetanic index in response to electrical stimulation upon treatment with compounds of the invention.
Figure 4 shows the increase of utrophin expression in human DMD culture of immortalized DMD patient-derived 3D skeletal muscle tissues upon treatment with compounds of the invention.

### DETAILED DESCRIPTION OF THE INVENTION

As outlined above, the present invention pertains to a series of nine compounds which have been found to induce utrophin upregulation. These compounds are presented in detail below.

Veliflapon (CAS 128253-31-6; https://pubchem.ncbi.nlm.nih.gov/compound/Veliflapon), also known as Bay-x-1005, is a compound of the following formula:

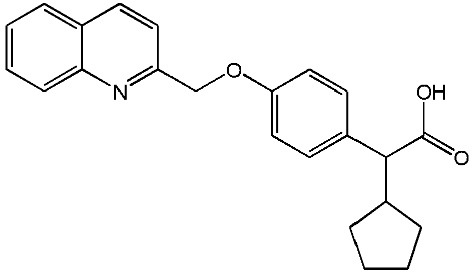

Preferably, Veliflapon refers to the following stereoisomer:

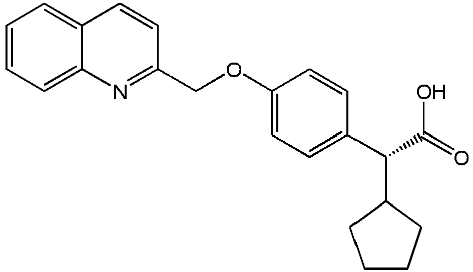

Veliflapon has been used for the treatment of acute coronary syndrome in patients with a history of unstable angina or myocardial infarction. The compound is commercially available, such as from Sigma Aldrich (Ref. SML0411); or may be synthesized using a suitable preparation method, such as that disclosed in US 4970215.

Difenpiramide (CAS 51484-40-3; https://pubchem.ncbi.nlm.nih.gov/compound/Difenpiramide), is a compound of the following formula:

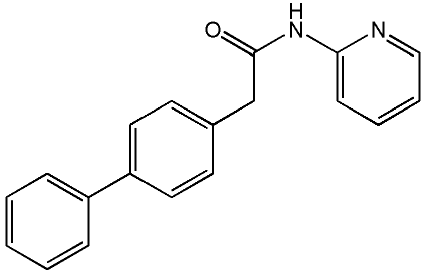

This compound has been used for the treatment of degenerative and inflammatory arthropathies such as osteoarthritis or rheumatoid arthritis. The compound is commercially available, such as from Smolecule (Ref.: S526059). The compound may also be synthesized by condensation of the carboxy group ofiphenyl-4-ylacetic acid (CAS 5728-52-9) with the amino group of 2-aminopyridine (CAS 504-29-0), both also readily available reagents, such as from Sigma-Aldrich (Refs.: 196487 and 09340, respectively). The compound may also be synthesized by the method disclosed in US 3868380 or in Khullar, Asian J. Chem., Vol. 31, No. 3 (2019), 510-514.

Amopyroquine (CAS 550-81-2; https://pubchem.ncbi.nlm.nih.gov/compound/Amopyroquine); is a compound of the following formula:

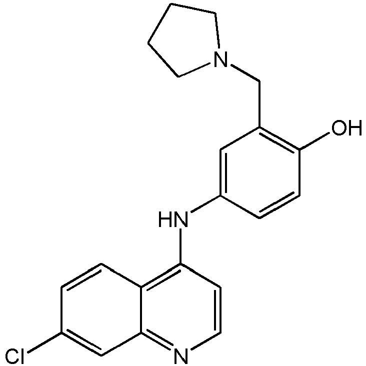

This compound has been used as an antimalarial agent. The compound is commercially available, such as from Smolecule (Ref. S518739); or may be synthesized using a suitable preparation method, such as that disclosed in Nobles et al., Journal of Pharmaceutical Sciences, Volume 52, Issue 6, June 1963, Pages 600-601.

Cloricromen (CAS 68206-94-0; https://pubchem.ncbi.nlm.nih.gov/compound/Cloricromen) is a compound of the following formula:

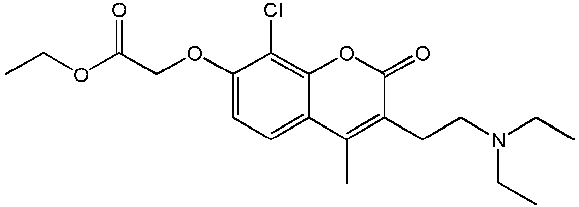

This compound has been used as an antithrombotic agent, specifically for the treatment of arterial thrombosis. The compound is commercially available, such as from Parchem (Ref.: 28592); or may be synthesized using a suitable preparation method, such as that disclosed in US 4452811 A.

Alpelisib (CAS 1217486-61-7; https://pubchem.ncbi.nlm.nih.gov/compound/Alpelisib), also known as BYL-719, is a compound of the following formula:

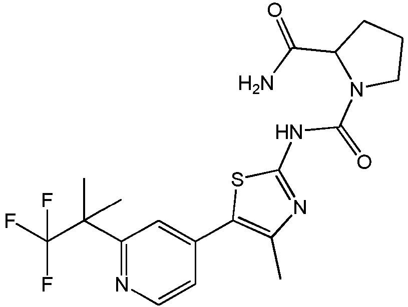

Preferably, Alpelisib refers to the following stereoisomer:

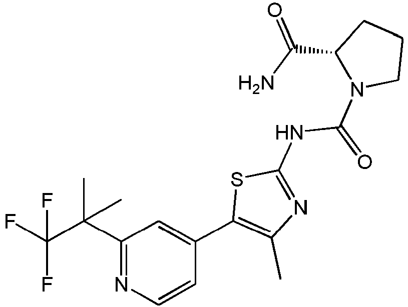

Alpelisib has been used as an antineoplastic agent, specifically for the treatment of metastatic breast cancer. The compound is commercially available, such as from Abcr GmbH (Ref.: AB337610); or may be synthesized using a suitable preparation method, such as that disclosed in US2010105711 A1.

Benorilate (CAS 5003-48-5; https://pubchem.ncbi.nlm.nih.gov/compound/Benorilate) is a compound of the following formula:

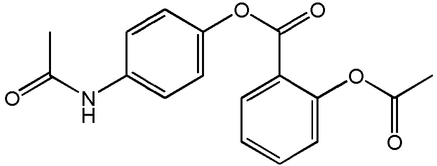

This compound has been used as an anti-inflammatory, antipyretic and analgesic agent. The compound is commercially available, such as from Sigma-Aldrich (Ref.: PH003407); or may be synthesized using a suitable preparation method, such as that disclosed in US3431293.

Xipamide (CAS 14293-44-8; https://pubchem.ncbi.nlm.nih.gov/compound/26618) is a compound of the following formula:

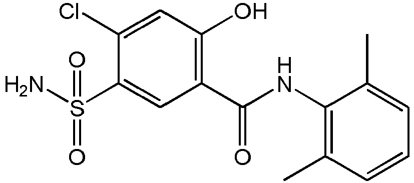

This compound has been used as a diuretic for the treatment of edema and hypertension. The compound is commercially available, such as from Parchem (Ref.: 13928); or may be synthesized using a suitable preparation method, such as that disclosed in US 3567777.

Fabomotizole (CAS 173352-21-1; https://pubchem.ncbi.nlm.nih.gov/compound/Fabomotizole), also known as Afobazole, is a compound of the following formula:

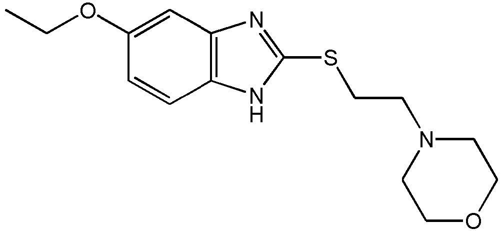

This compound has been used as an anxiolytic. The compound is commercially available, such as from Parchem (Ref.: 110780); or may be synthesized using a suitable preparation method, such as that disclosed in US6376666 B1.

Altizide (CAS 5588-16-9; https://pubchem.ncbi.nlm.nih.gov/compound/Althiazide), also known as Althiazide, is a compound of the following formula:

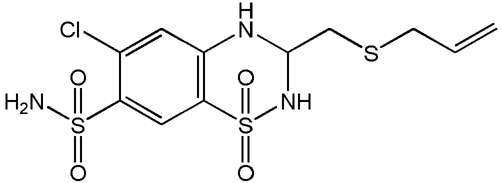

Preferably, Altizide refers to the following stereoisomer:

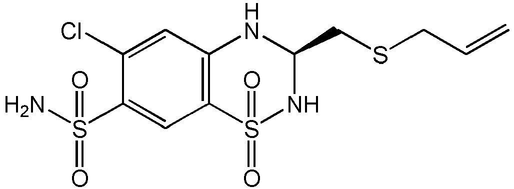

Altizide has been used as a diuretic for the treatment of edema, hypertension and hormonal disorders. The compound is commercially available, such as from Sigma-Aldrich (Ref.: Y0000606); or may be synthesized using a suitable preparation method, such as that disclosed in US 3111517; and enantioseparated such as by the method reported in Zhong et al., J. Chromatogr. A 1115 (2006) 19-45.

The above compounds and their salts as described below may also be purchased from alternative chemical vendors, such as those found in the PubChem database maintained by the National Center for Biotechnology Information (NCBI), as can for instance be verified at the different https://pubchem.ncbi.nlm.nih.gov webpages provided herein.

In a preferred embodiment, the compound is selected from Difenpiramide, Veliflapon, Cloricromen, Alpelisib, Fabomotizole, or a pharmaceutically acceptable salt thereof.

In a preferred particular embodiment, the compound is Veliflapon, or a pharmaceutically acceptable salt thereof, preferably it is Veliflapon. A preferred Veliflapon salt is the sodium salt, which is commercially available, such as from THE BioTek (Ref. bt-285834); or may be prepared using a suitable preparation method, such as that disclosed in US 4970215.

In a preferred particular embodiment, the compound is Difenpiramide or a pharmaceutically acceptable salt thereof, preferably Difenpiramide. Preferred Difenpiramide salts are acid addition salts, such as the hydrochloride, sulphate, succinate or maleate salt thereof, which are described in US 3868380.

In a particular embodiment, the compound is Amopyroquine, or a pharmaceutically acceptable salt thereof, preferably it is Amopyroquine. A preferred Amopyroquine salt is a hydrochloride salt, more preferably it is the dihydrochloride salt, which is commercially available, such as from Smolecule (Ref. S518740); or may be prepared using a suitable preparation method, such as that disclosed in Nobles et al., Journal of Pharmaceutical Sciences, Volume 52, Issue 6, June 1963, Pages 600-601.

In a preferred particular embodiment, the compound is Cloricromen, or a pharmaceutically acceptable salt thereof, preferably it is the pharmaceutically acceptable salt thereof. A preferred Cloricromen salt is the hydrochloride salt, which is commercially available, such as from Sigma-Aldrich (Ref. C4615); or may be prepared using a suitable preparation method, such as that disclosed in US 4452811 A.

In a preferred particular embodiment, the compound is Alpelisib, or a pharmaceutically acceptable salt thereof, preferably it is Alpelisib. Preferred Alpelisib salts are acid addition salts, such as the hydrochloride salt, which are described in US2010105711 A1 or are commercially available such as from AdooQ Bioscience (Ref.: A21794).

In a particular embodiment, the compound is Benorilate, or a pharmaceutically acceptable salt thereof, preferably it is Benorilate. Benorilate salts, such as the hydrochloride salt thereof, are commercially available such as from AKos Consulting & Solutions GmbH (Ref.: AKOS015960742).

In a particular embodiment, the compound is Xipamide, or a pharmaceutically acceptable salt thereof, preferably it is Xipamide. Preferred Xipamide salts are ammonium and alkali metal salts, such as those disclosed in US 3567777.

In a preferred particular embodiment, the compound is Fabomotizole, or a pharmaceutically acceptable salt thereof, preferably it is the pharmaceutically acceptable salt thereof. A preferred Fabomotizole salt is the hydrochloride salt (CAS 173352-39-1, 189638-30-0; https://pubchem.ncbi.nlm.nih.gov/compound/Afobazol). Another preferred salt is the dihydrochloride salt. These salts are commercially available, such as from Smolecule (Ref.: S517427); or may be prepared using a suitable preparation method, such as that disclosed in US6376666 B1.

In a particular embodiment, the compound is Althiazide or a pharmaceutically acceptable salt thereof, preferably it is Althiazide. Preferred Althiazide salts are base addition salts such as the potassium salt, which are described in US 3111517.

The term "pharmaceutically acceptable" refers to molecular entities and compositions that are physiologically tolerable and do not typically produce an allergic or similar untoward reaction, such as gastric upset, dizziness and the like, when administered to a human.

Further, the term "pharmaceutically acceptable salt" refers to any salt, which, upon administration to the recipient is capable of providing (directly or indirectly) a compound as described herein. For instance, pharmaceutically acceptable salts of compounds provided herein may be acid addition salts or base addition salts, and they can be synthesized from the parent compound, which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, non-aqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate. Examples of the alkali addition salts include organic salts such as for example, ammonium salts, or inorganic salts such as alkali metal salts such as sodium or potassium salts.

The combinations of the present invention are combinations of Difenpiramide, Veliflapon, Amopyroquine, Cloricromen, Alpelisib, Benorilate, Xipamide, Fabomotizole and Altizide, either with each other or with other compounds known to be useful in the treatment and/or prevention of a dystrophinopathy. The present invention, for the first time, provides the motivation to produce said combinations.

In a preferred embodiment, the combination is a combination comprising two or more agents selected from the group consisting of Difenpiramide, Veliflapon, Amopyroquine, Cloricromen, Alpelisib, Benorilate, Xipamide, Fabomotizole, Altizide, or a pharmaceutically acceptable salt thereof; ataluren, deflazacort, givinostat, pizuglanstat, ifetroban, vamorolone, idebenone, prednisone, prednisolone, 20-hydroxyecdysone, metformin and L-citrulline combination, MA-0211, tamoxifen, dextro epicatechin, exaluren, ARM-210, 2,6-disubstituted-pyiridazin-3(2H)-ones, N-Benzyl-p-toluenesulphonamide, MP-101, MT-002, rimeporide, tetracosactide hexaacetate, elamipretide, KER-050, SRP-5051, TXA-127, PB-1023, pamrevlumab, eteplirsen, golodirsen, vitolarsen, casimersen, ATL-1102, NS-089, renadirsen, WVEN-531, fordadistrogene movaparvovec, GNT-004, AAV1-follistatin viral vector, delandistrogene moxeparvovec, AT-702, AAVrh74-GALGT2, SGT-001, CAP-2003, CAP-1002; wherein at least one of said agents is selected from the group consisting of Difenpiramide, Veliflapon, Amopyroquine, Cloricromen, Alpelisib, Benorilate, Xipamide, Fabomotizole and Altizide, or a pharmaceutically acceptable salt thereof.

**Ataluren** (CAS 775304-57-9; https://pubchem.ncbi.nlm.nih.gov/compound/11219835) is a compound of the following formula:

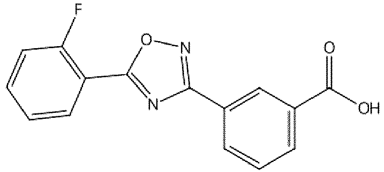

This compound enables ribosomal read-through of mRNA containing premature stop codons and is commercialized under the brand name Translarna for the treatment of DMD. The compound is commercially available, such as from MedChemExpress (Ref.: HY-14832); or may be synthesized using a suitable preparation method, such as that disclosed in US20040204461.

**Deflazacort** (CAS 14484-47-0; https://pubchem.ncbi.nlm.nih.gov/compound/189821) is a compound of the following formula:

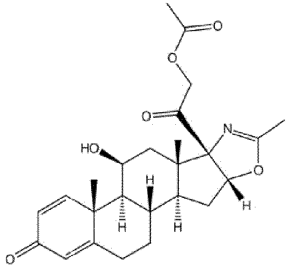

This compound is a glucocorticoid commercialized under the brand name Emflaza for the treatment of DMD. The compound is commercially available, such as from MedChemExpress (Ref.: HY-13609); or may be synthesized using a suitable preparation method, such as that disclosed in WO9721722.

**Givinostat** (CAS 497833-27-9; https://pubchem.ncbi.nlm.nih.gov/compound/9804992) is a compound of the following formula:

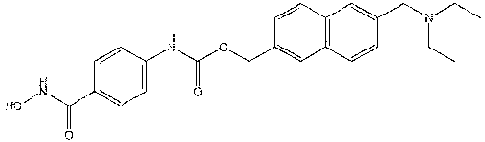

This compound is a histone deacetylase inhibitor currently in development for the treatment of DMD. The compound is commercially available, such as from MedChemExpress (Ref.: HY-14842); or may be synthesized using a suitable preparation method, such as that disclosed in WO2020178776.

**Pizuglanstat,** also known as TAS-205 (CAS 1244967-98-3; https://pubchem.ncbi.nlm.nih.gov/compound/46911296) is a compound of the following formula:

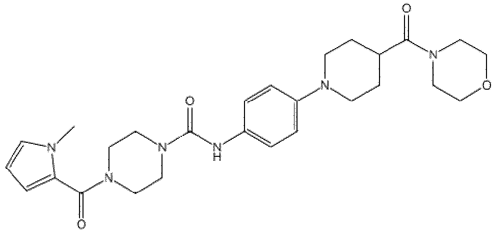

This compound is a selective prostaglandin D2 synthase inhibitor currently in development for the treatment of DMD. The compound is commercially available, such as from MedChemExpress (Ref.: HY-109134); or may be synthesized using a suitable preparation method, such as that disclosed in US8865714.

**Ifetroban** (CAS 143443-90-7; https://pubchem.ncbi.nlm.nih.gov/compound/3037233) is a compound of the following formula:

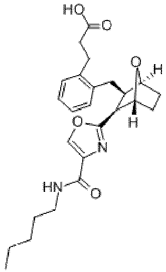

This compound is a thromboxane A2/prostaglandin endoperoxide (TP) receptor antagonist currently in development for the treatment of DMD. The compound is commercially available, such as from BOC Sciences (Ref.: 143443-90-7); or may be synthesized using a suitable preparation method, such as that disclosed in US5332840.

**Vamorolone,** also known as VBP-15 (CAS 13209-41-1; https://pubchem.ncbi.nlm.nih.gov/compound/3035000) is a compound of the following formula:

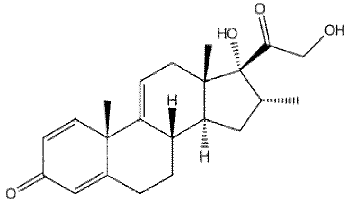

This compound is a glucocorticoid analog currently in development for the treatment of DMD. The compound is commercially available, such as from MedChemExpress (Ref.: HY-109017); or may be synthesized using a suitable preparation method, such as that disclosed in US9198921.

**Idebenone** (CAS 58186-27-9; https://pubchem.ncbi.nlm.nih.gov/compound/3686) is a compound of the following formula:

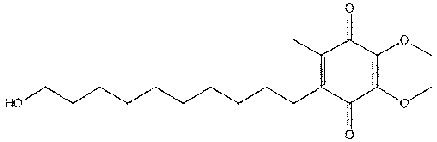

This compound is a coenzyme Q10 analog and an antioxidant analog that is in clinical trials for the treatment of DMD. The compound is commercially available, such as from MedChemExpress (Ref.: HY-N0303); or may be synthesized using a suitable preparation method, such as that disclosed in EP21841.

**Prednisone** (CAS 53-03-2; https://pubchem.ncbi.nlm.nih.gov/compound/5865) is a compound of the following formula:

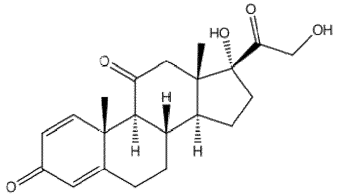

This compound is a glucocorticoid analog used for the treatment of DMD. The compound is commercially available, such as from MedChemExpress (Ref.: HY-B0214); or may be synthesized using a suitable preparation method, such as that disclosed in GB1063821.

**Prednisolone** (CAS 50-24-8; https://pubchem.ncbi.nlm.nih.gov/compound/5755) is a compound of the following formula:

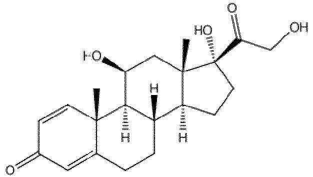

This compound is a glucocorticoid analog used for the treatment of DMD. The compound is commercially available, such as from MedChemExpress (Ref.: HY-17463); or may be synthesized using a suitable preparation method, such as that disclosed in EP127294.

**20-Hydroxyecdysone,** also known as BIO-101 or crustecdysone (CAS 5289-74-7; https://pubchem.ncbi.nlm.nih.gov/compound/5459840), is a compound of the following formula:

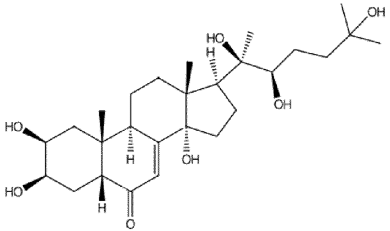

The compound is an ecdysteroid that is in clinical development for the treatment of DMD. The product is commercially available, such as from MedChemExpress (Ref.: HY-N6979), or may be synthesized using a suitable preparation method, such as that disclosed in WO2012053994.

**Metformin + L-citrulline** is a combination of metformin (CAS 657-24-9; https://pubchem.ncbi.nlm.nih.gov/compound/4091), a compound of the following formula: 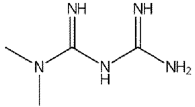 and L-citrulline (CAS 372-75-8; https://pubchem.ncbi.nlm.nih.gov/compound/9750), a compound of the following formula:

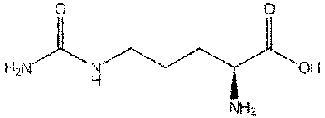

The combination Metformin + L-citrulline is in clinical trials for the treatment of DMD. Metformin is commercially available, such as from MedChemExpress (Ref.: HY-B0627), or may be synthesized using a suitable preparation method, such as that disclosed in WO2016059507. L-citrulline is commercially available, such as from MedChemExpress (Ref.: HY-N0391), or may be synthesized using a suitable preparation method, such as that disclosed in GB1176266.

**MA-0211** (also known as ASP-0367; MTB-1; bocidelpar sulfate), is a compound of the following formula:

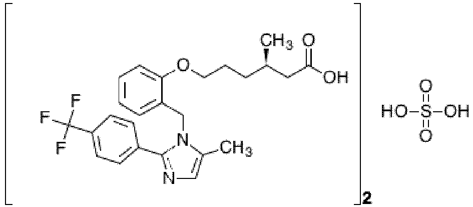

This compound is a small molecule acting as modulator of PPAR-delta. The product is in clinical development by Mitobridge for the treatment of DMD and mitochondrial diseases, and may be synthesized using a suitable preparation method, such as that disclosed in WO2017062468 A1.

**Tamoxifen** (CAS 10540-29-1; https://pubchem.ncbi.nlm.nih.gov/compound/2733526), is a compound of the following formula:

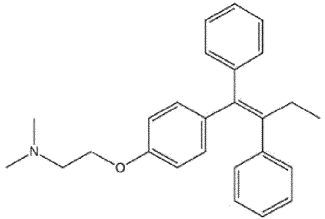

The compound is a nonsteroidal selective estrogen receptor modulator that is in clinical development for the treatment of DMD. The product is commercially available, such as from MedChemExpress (Ref.: HY-13757A), or may be synthesized using a suitable preparation method, such as that disclosed in US4536516.

**Dextro epicatechin,** also known as (+)-epicatechin (CAS 35323-91-2; https://pubchem.ncbi.nlm.nih.gov/compound/182232), is a compound of the following formula:

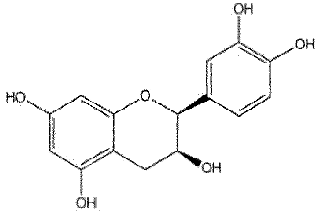

The compound is a polyphenol derived from green tea that is in clinical development for the treatment of DMD. The compound is commercially available, such as from MedChemExpress (Ref.: HY-N0001A), or may be synthesized using a suitable preparation method, such as that disclosed in WO2014115174.

**Exaluren** (CAS 1375073-93-0; https://pubchem.ncbi.nlm.nih.gov/compound/71461382), is a compound of the following formula:

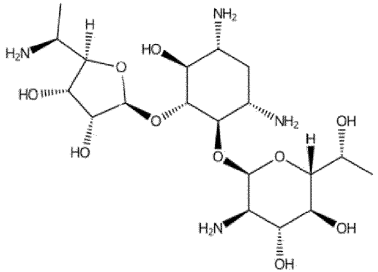

The compound is an aminoglycoside that is being developed as a disulfate salt for the treatment of DMD and other diseases. The compound is commercially available, such as from MedChemExpress (Ref.: HY-114231), or may be synthesized using a suitable preparation method, such as that disclosed in US8895519.

**ARM-210** (CAS 1467605-57-7; https://pubchem.ncbi.nlm.nih.gov/compound/71761628), is a compound of the following formula:

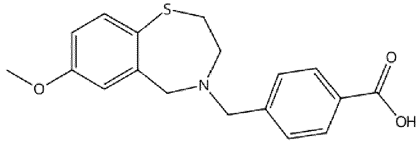

This compound is a ryanodine receptor/calcium release channel stabilizer that is currently in clinical development for the treatment of DMD. The compound is commercially available, such as from Chemieliva (Ref.: CB1384540), or may be synthesized using a suitable preparation method, such as that disclosed in US8853198.

**2,6-disubstituted-pyiridazin-3(2H)-ones** suitable for the treatment of DMD have been described in the art and are in clinical development for the treatment of DMD. Examples of suitable 2,6-disubstituted-pyiridazin-3(2H)-ones are those described in WO2021231630. A particular suitable example is Compound 5 of WO2021231630.

**N-Benzyl-p-toluenesulphonamide**, also known as BTS (CAS 1576-37-0; https://pubchem.ncbi.nlm.nih.gov/compound/N-Benzyl-p-toluenesulfonamide), is a compound of the following formula:

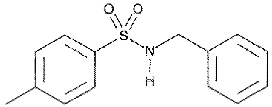

This compound is an inhibitor of fast-fiber skeletal muscle myosin and has been shown to protect muscles from pathological muscle derangement in DMD animal models. The compound is commercially available, such as from Sigma-Aldrich (Ref.: 203895).

**MP-101** (also known as 2,4-dinitrophenol) (CAS 51-28-5; https://pubchem.ncbi.nlm.nih.gov/compound/1493), is a compound of the following formula:

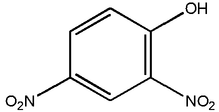

The compound acts as mitochondria modulator and is in clinical development for the treatment of DMD. The compound is commercially available, such as from Sigma-Aldrich (Ref.: 07-5190), or may be synthesized using a suitable preparation method, such as that disclosed in Khabarov et al., Russian Journal of Applied Chemistry, 2012, Vol. 85, No. 10, pp. 1577-1580.

**MT-002,** also known as AZD-9056 or MP-118, (CAS 345304-65-6; https://pubchem.ncbi.nlm.nih.gov/compound/1493), is a compound of the following formula:

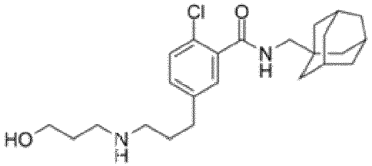

It is an antagonist of the P2X7 channel that is in clinical development for the treatment of DMD. The compound is commercially available, such as from Smolecule (Ref.: S520142), or may be synthesized using a suitable preparation method, such as that disclosed in US7297818.

**Rimeporide** (CAS 187870-78-6; https://pubchem.ncbi.nlm.nih.gov/compound/9799487), also known as EMD-87580, is a compound of the following formula:

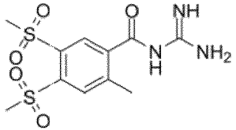

It is an inhibitor of Na+ H+ exchange-1 (NHE-1) channel. This product is in clinical development for the treatment of DMD. The compound is commercially available, such as from MedChemExpress (Ref.: HY-19273), or may be synthesized using a suitable preparation method, such as that disclosed in US5744641.

**Tetracosactide hexaacetate** (CAS 22633-88-1; https://pubchem.ncbi.nlm.nih.gov/compound/Tetracosactide-hexaacetate) also known as Cosyntropin Depot, Synacthen Depot or MNK-1411, is a synthetic 24-amino acid melanocortin receptor agonist and analog of adrenocorticotrophic hormone (ACTH). The product is in clinical development by Mallinckrodt for the treatment of DMD. The product can be obtained by any suitable preparation method, such as that disclosed in US2015110885.

**Elamipretide** (CAS 736992-21-5; https://pubchem.ncbi.nlm.nih.gov/compound/11764719) is a synthetic peptide targeting cardiolipin and it is in clinical development for the treatment of DMD and other diseases. The product is commercially available, such as from Carbosynth (Ref.: LEB99221), or can be obtained by any suitable preparation method, such as that disclosed in US9988422.

**KER-050** is a fusion protein acting as TGF beta inhibitor that is in clinical development by Keros Therapeutics for the treatment of DMD. This product can be obtained by a suitable preparation method, such as that disclosed in WO2018089706. In fact, any polypeptide as defined in claim 1 of WO2018089706, which is herein incorporated by reference, can be used as the agent in the combination of the invention.

**SRP-5051** is a peptide phosphorodiamidate morpholino oligomer compound, comprising eteplirsen conjugated to a cell-penetrating peptide, as an exon-skipping therapy targeting exon 51 of dystrophin. This product is in clinical development for the treatment of DMD. This product can be obtained by a suitable preparation method, such as that disclosed in WO2019079386. In fact, any peptide-oligonucleotide conjugate as defined in claim 1 of WO2019079386, which is herein incorporated by reference, can be used as the agent in the combination of the invention.

**TXA-127** is angiotensin II 1-7 (CAS 39386-80-6; https://pubchem.ncbi.nlm.nih.gov/compound/123805). This product is a naturally-occurring small peptide that is in clinical development for the treatment of DMD. This product is commercially available from MedChemExpress (Ref HY-12403).

**PB-1023,** also known as Glymera (CAS 1417579-81-7) is a long acting glucagon like peptide-1 (GLP-1) elastin like peptide (ELP)-120 fusion protein that acts by targeting glucagon-like peptide 1 receptor (GLP1R). This product is in clinical development for the treatment of DMD. This product can be obtained by a suitable preparation method, such as disclosed in WO2014113434 A1.

**Pamrevlumab,** also known as FG-3019 (CAS 946415-13-0), is a recombinant human IgG1/kappa monoclonal antibody against connective tissue growth factor (CTGF; CCN2). The product is in clinical development for the treatment of DMD and other fibrotic diseases. The product is commercially available, such as from Creative Biolabs (Ref.: TAB-443CQ).

**Eteplirsen** (CAS 1173755-55-9) is an antisense 30-mer phosphorodiamidate morpholino oligonucleotide targeted to skip dystrophin gene exon 51. The product is commercialized under the trade name Exondys 51 for the treatment of DMD patients with mutations amenable to exon 51 skipping. The product may be synthesized using a suitable preparation method, such as that disclosed in WO2017205879.

**Golodirsen** (CAS 1422959-91-8) is an antisense phosphorodiamidate morpholino oligomer-based RNA therapeutic, targeted to skip dystrophin gene exon 53. The product is commercialized under the trade name Vyondys 53 for the treatment of DMD patients with mutations amenable to exon 53 skipping. The product may be synthesized using a suitable preparation method, such as that disclosed in WO2021025899.

**Viltolarsen** (CAS 2055732-84-6) is morpholino-based antisense oligonucleotide designed to skip dystrophin gene exon 53. The product is commercialized under the trade name Viltepso for the treatment of DMD patients with mutations amenable to exon 53 skipping. The compound is commercially available, such as from MedChemExpress (Ref.: HY-132586).

**Casimersen** (CAS 1422958-19-7) is an antisense phosphorodiamidate morpholino oligomer -based antisense RNA therapeutic, targeted to skip dystrophin gene exon 45. The product is commercialized under the trade name Amondys 45 for the treatment of DMD patients with mutations amenable to exon 45 skipping. The product may be synthesized using a suitable preparation method, such as that disclosed in WO2017205879.

**ATL-1102,** also known as TV-1102 or ISIS-107248 (CAS 350263-41-1), is a second-generation antisense oligonucleotide inhibiting CD49d (a subunit of VLA-4) mRNA (ITGA4 gene). The product is in clinical development by Antisense Therapeutics for the treatment of DMD. The product is described in Limmroth et al., Neurology. 2014 Nov 11; 83(20): 1780-1788 and may be synthesized using a suitable preparation method, such as that disclosed in US6258790.

**NS-089,** also known as NCNP-02, is an exon-skipping antisense oligonucleotide targeting exon 44 of dystrophin gene. This product is in clinical development for the treatment of DMD. The product may be synthesized using a suitable preparation method, such as that disclosed in EP3660154. In fact, any antisense oligomer as defined in claim 1 of EP3660154 A1, which is herein incorporated by reference, can be used as the agent in the combination of the invention.

**Renadirsen,** also known as DS-5141 (CAS 1782108-31-9), is an oligonucleotide therapy which induces exon 45 skipping. This product is in clinical development for the treatment of DMD. The product may be synthesized using a suitable preparation method, such as that disclosed in Ito et al. 2021 Current issues in molecular biology; 43 (3): 1267-1281 (DOI: 10.3390/cimb43030090).

**WVE-N531** is a phosphorothioate modified nucleic acid which induces exon 53 skipping in the dystrophin gene. This product is in clinical development for the treatment of DMD. The product may be synthesized using a suitable preparation method, such as that disclosed in WO2019217784. In fact, any oligonucleotide as defined in claims 1 to 5 of WO2019217784, which is herein incorporated by reference, can be used as the agent in the combination of the invention.

**Fordadistrogene movaparvovec,** also known as PF-06939926 or BMB-D001 (CAS 2248111-37-5), is a gene therapy agent that uses a recombinant adeno-associated viral vector serotype 9 (AAV9) to deliver a functional mini-dystrophin gene. The product is in clinical development by Pfizer for the treatment of DMD. The product may be synthesized using a suitable preparation method, such as that disclosed in CA2971303.

**GNT-004** is an adeno-associated viral vector 8 (AAV8) gene therapy agent encoding a microdystrophin gene. The product is in clinical development by Genethon for the treatment of DMD. The product may be synthesized using a suitable preparation method, such as that disclosed in WO2015197232. Any composition as defined in claim 1 of WO2015197232, which is herein incorporated by reference, can be used as the agent in the combination of the invention.

**AAV1-follistatin viral vector** is an adeno-associated virus (AAV) gene therapy agent delivering a muscle-specific transgene of follistatin, a myostatin and TGF beta ligand inhibitor. The product is in clinical development by Milo Biotechnology for the treatment of DMD. The product may be synthesized using a suitable preparation method, such as that disclosed in WO2008067480. Any composition as defined in claim 1 of WO2008067480, which is herein incorporated by reference, can be used as the agent in the combination of the invention.

**Delandistrogene moxeparvovec,** also known as SRP-9001 or RG-6356 (CAS 2305040-16-6), is a gene therapy agent that uses an adeno-associated virus serotype rh74 containing the human micro-dystrophin gene. The product is in clinical development by Sarepta Therapeutics for the treatment of DMD. The product may be synthesized using a suitable preparation method, such as that disclosed in WO2021108755. Any of the vectors described in the invention of WO2021108755, which is herein incorporated by reference, can be used as the agent in the combination of the invention.

**AT-702** is a recombinant adeno-associated virus 9 (AAV9) vector containing 4 antisense sequences that induce exon 2 skipping in the human DMD gene. The product is in clinical development by Astellas for the treatment of DMD. The product may be synthesized using a suitable preparation method, such as that disclosed in WO2014172669. Any recombinant adeno-associated virus as defined in claim 1 of WO2014172669, which is herein incorporated by reference, can be used as the agent in the combination of the invention.

**AAVrh74-GALGT2** is a gene therapy agent that uses a recombinant adeno-associated virus serotype rhesus 74 (rh74) expressing the human CT GalNac transferase (GALGT2) gene. The product is in clinical development by Sarepta Therapeutics for the treatment of DMD. The product may be synthesized using a suitable preparation method, such as that disclosed in WO2017049031. Any recombinant adeno-associated virus as defined in claim 1 of WO2017049031, which is herein incorporated by reference, can be used as the agent in the combination of the invention.

**SGT-001** is an adeno-associated virus serotype 9 (AAV9) vector-based microdystrophin gene therapy agent that is in development for the treatment of DMD. The product is described and may be synthesized as disclosed in Bostick et al. 2008 Human Gene Therapy 19:851-856 (DOI: 10.1089/hum.2008.058)

**CAP-2003** refers to extracellular vesicles, including exosomes, which are derived from cardiosphere-derived cells (CDC-EVs). These vesicles deliver micro-RNA and elicit cardioprotective and muscular regenerative properties. This product is in clinical development for the treatment of DMD. The product is described and may be synthesized as disclosed in WO2018195210, which is herein incorporated by reference.

**CAP-1002** is a stem cell treatment developed by Capricor Therapeutics Inc for the treatment of DMD. The product may be synthesized using a suitable preparation method, such as that disclosed in WO2013184527. Any cardiosphere-derived cells as defined in claim 1 of WO2013184527, which is herein incorporated by reference, can be used as the agent in the combination of the invention.

In a preferred particular embodiment, at least one of said agents comprised in the combination is Veliflapon or a pharmaceutically acceptable salt thereof, as presented in any of the embodiments described above.

In a preferred particular embodiment, at least one of said agents comprised in the combination is Difenpiramide or a pharmaceutically acceptable salt thereof, as presented in any of the embodiments described above.

In a particular embodiment, at least one of said agents comprised in the combination is Amopyroquine or a pharmaceutically acceptable salt thereof, as presented in any of the embodiments described above.

In a preferred particular embodiment, at least one of said agents comprised in the combination is Cloricromen or a pharmaceutically acceptable salt thereof, as presented in any of the embodiments described above.

In a preferred particular embodiment, at least one of said agents comprised in the combination is Alpelisib or a pharmaceutically acceptable salt thereof, as presented in any of the embodiments described above.

In a particular embodiment, at least one of said agents comprised in the combination is Benorilate or a pharmaceutically acceptable salt thereof, as presented in any of the embodiments described above.

In a particular embodiment, at least one of said agents comprised in the combination is Xipamide or a pharmaceutically acceptable salt thereof, as presented in any of the embodiments described above.

In a preferred particular embodiment, at least one of said agents comprised in the combination is Fabomotizole or a pharmaceutically acceptable salt thereof, as presented in any of the embodiments described above.

In a particular embodiment, at least one of said agents comprised in the combination is Althiazide or a pharmaceutically acceptable salt thereof, as presented in any of the embodiments described above.

In an embodiment, the other one of the two or more agents comprised in the combination is a compound, such as ataluren, deflazacort, givinostat, pizuglanstat, ifetroban, vamorolone, idebenone, prednisone, prednisolone, 20-hydroxyecdysone, metformin and L-citrulline combination, MA-0211, tamoxifen, dextro epicatechin, exaluren, ARM-210, 2,6-disubstituted-pyiridazin-3(2H)-ones, N-Benzyl-p-toluenesulphonamide, MP-101, MT-002, or rimeporide.

In another embodiment, the other one of the two or more agents comprised in the combination is a biological agent comprising a peptide or a protein, such as tetracosactide hexaacetate, elamipretide, KER-050, SRP-5051, TXA-127, or PB-1023, wherein the protein may in particular be an antibody, such as pamrevlumab; a nucleic acid, such as eteplirsen, golodirsen, vitolarsen, casimersen, ATL-1102, NS-089, renadirsen, or WVEN-531, wherein the nucleic acid may be administered by employing a viral vector, such as fordadistrogene movaparvovec, GNT-004, AAV1-follistatin viral vector, delandistrogene moxeparvovec, AT-702, AAVrh74-GALGT2, or SGT-001; or cells and material obtained therefrom, such as CAP-2003 or CAP-1002.

The present invention is directed to the medical use of said compounds and combinations in the treatment and/or prevention of a dystrophinopathy. The term "dystrophinopathy", as used in the present invention, designates any disease or disorder caused by an absence or deficiency of dystrophin. Non-limiting examples of a disease or disorder caused by an absence or deficiency of dystrophin are DMD, BMD, DMD-associated dilated cardiomyopathy or cramps with myoglobinuria. Preferably, the disease or disorder caused by an absence or deficiency of dystrophin is selected from DMD and BMD, and even more preferably it is DMD.

The terms "treating" and "treatment", as used herein, means reversing, alleviating, inhibiting the progress of the disease or disorder, or of one or more symptoms of said disease or disorder such as muscle weakness, enlargement of the heart, limited breathing, scoliosis, joint contractures, calf muscle hypertrophy or cognitive impairment, with respect to pretreatment levels.

The terms "preventing" and "prevention", as used herein, means avoiding or inhibiting the onset of said disease or disorder, or of one or more of said symptoms.

The compounds for use according to the invention may be administered by any appropriate route. Non-limiting examples of administration routes are the oral, parenteral (e.g., subcutaneous, intramuscular, intravenous, etc.), topical, sublingual, inhaled, rectal, vaginal, or ophthalmic routes. In a preferred embodiment, the compounds for use according to the invention are administered by oral or parenteral route, the intravenous and intramuscular routes being a particularly preferred parenteral routes.

In particular, the compounds for use according to the invention are administered as a pharmaceutical composition, which comprises the corresponding active compound(s) and one or more pharmaceutically acceptable excipients. The term "pharmaceutically acceptable excipient" refers to a vehicle, diluent, or adjuvant that is administered with the active ingredient. The identity of the pharmaceutically acceptable excipient will necessarily depend, among other factors, on the elected administration route. Said pharmaceutical compositions may be manufactured according to conventional methods known by the skilled person in the art, such as those described in Spanish and US Pharmacopoeias.

Thus, the present invention also relates to a pharmaceutical composition comprising a compound selected from the group consisting of Difenpiramide, Veliflapon, Amopyroquine, Cloricromen, Alpelisib, Benorilate, Xipamide, Fabomotizole and Altizide, or a pharmaceutically acceptable salt thereof, or to a combination according to the present invention; and at least one pharmaceutically acceptable excipient; for use in the treatment or prevention of a dystrophinopathy.

Similarly, the invention also relates to the use of a pharmaceutical composition comprising a compound selected from the group consisting of Difenpiramide, Veliflapon, Amopyroquine, Cloricromen, Alpelisib, Benorilate, Xipamide, Fabomotizole and Altizide, or a pharmaceutically acceptable salt thereof, or to a combination according to the present invention; and at least one pharmaceutically acceptable excipient; in the manufacture of a medicament for the treatment and/or prevention of a dystrophinopathy.

Similarly, the invention also relates to a method of treating and/or preventing a dystrophinopathy in a subject, comprising administering to said subject a therapeutically effective amount of a pharmaceutical composition comprising a compound selected from the group consisting of Difenpiramide, Veliflapon, Amopyroquine, Cloricromen, Alpelisib, Benorilate, Xipamide, Fabomotizole and Altizide, or a pharmaceutically acceptable salt thereof, or of a combination according to the present invention; and at least one pharmaceutically acceptable excipient.

In yet an additional aspect, the invention also relates to a pharmaceutical composition comprising a combination of the invention; and at least one pharmaceutically acceptable excipient.

In an embodiment, the pharmaceutical compositions are in oral delivery form. Pharmaceutical forms suitable for oral administration may be tablets and capsules and may contain conventional excipients known in the art such as binders, for example syrup, gum arabic, gelatin, sorbitol, tragacanth or polyvinylpyrrolidone; fillers, for example lactose, sugar, cornstarch, calcium phosphate, sorbitol or glycine; lubricants for the preparation of tablets, for example magnesium stearate; disintegrants, for example starch, polyvinylpyrrolidone, sodium starch glycolate or microcrystalline cellulose; or pharmaceutically acceptable wetting agents such as sodium lauryl sulphate. Solid oral compositions can be prepared by conventional methods such as blending, filling or compressing. Alternatively, pharmaceutical forms suitable for oral administration may be in the form of a solution or suspension, in particular wherein a powder or granules comprising the compound or combination of the invention are dissolved or dispersed in a liquid medium. Oral solutions or suspensions are particularly preferred when administration is to a pediatric human subject.

In an embodiment, the pharmaceutical compositions are in parenteral, preferably intravenous or intramuscular, delivery form.

Pharmaceutical forms suitable for parenteral administration may be sterile solutions or suspensions based on aqueous vehicles such as water or sodium chloride, Ringer or dextrose solutions; water-miscible vehicles including, but not limited to, ethyl alcohol, polyethylene glycol and polypropylene glycol; or non-aqueous vehicles including, but not limited to, corn oil, cottonseed oil, peanut oil, sesame oil, ethyl oleate, isopropyl myristate and benzyl benzoate. Suitable excipients such as fillers, buffering agents or surfactants can be used.

The compounds for use according to the invention are preferably administered in or comprised in the pharmaceutical compositions or combinations in a "therapeutically effective amount", i.e. a nontoxic but sufficient amount of the corresponding compound(s) to provide the desired effect, namely the treatment and/or prevention of a dystrophinopathy. The amount that is "effective" will vary from subject to subject, depending on the age and general condition of the individual, the particular compound administered, and the like. Thus, it is not always possible to specify an exact "therapeutically effective amount". However, an appropriate amount in any individual case may be determined by one of ordinary skill in the art using routine knowledge and experimentation.

The term "subject" preferably refers to a mammal, such as a human. In a preferred embodiment, in any embodiment described herein, the treatment or prevention of a dystrophinopathy is in a pediatric human subject. A pediatric human subject is one who is 21 years of age or younger, such as neonates (from birth through the first 28 days of life), infants (29 days to less than 2 years), children (2 years to less than 12 years), adolescents (12 years through 21 years, up to but not including the 22nd birthday).

The compounds for use according to the invention will typically be administered once or more times a day, for example 1, 2, 3 or 4 times daily, with typical total daily doses depending on a number of factors such as the particular compound to be administered, the severity of the disease or the weight or age of the patient (a good number of dystrophinopathy-suffering patients are pediatric), all of which are known to the skilled practitioner, who can then determine appropriate doses based on his or her common general knowledge.

Typical total daily doses of veliflapon, or a pharmaceutically acceptable salt thereof, are in the range of from 1 to 3000 mg/day (expressed as veliflapon free base), preferably from 30 to 1500 mg/day. The compound may be administered by oral route.

Typical total daily doses of difenpiramide, or a pharmaceutically acceptable salt thereof, are in the range of from 1 to 3000 mg/day (expressed as difenpiramide free base), preferably from 10 to 1500 mg/day. The compound may be administered by oral route.

Typical total daily doses of amopyroquine, or a pharmaceutically acceptable salt thereof, are in the range of from 0.1 to 250 mg/kg/ day (expressed as amopyroquine free base), preferably from 1 to 25 mg/kg/day. The compound may be administered by parenteral route, preferably by intramuscular route.

Typical total oral daily doses of cloricromen, or a pharmaceutically acceptable salt thereof, are in the range of from 1 to 1000 mg/day (expressed as cloricromen free base), preferably from 10 to 300 mg/day. Typical total intravenous daily doses of cloricromen, or a pharmaceutically acceptable salt thereof, are in the range of from 1 to 100 mg/day (expressed as cloricromen free base), preferably from 10 to 30 mg/day.

Typical total daily doses of alpelisib, or a pharmaceutically acceptable salt thereof, are in the range of from 1 to 2000 mg/day (expressed as alpelisib free base), preferably from 10 to 500 mg/day. The compound may be administered by oral route.

Typical total daily doses of benorilate, or a pharmaceutically acceptable salt thereof are in the range of from 0.05 to 50 g/day (expressed as benorilate free base), preferably from 0.5 to 20 g/day. The compound may be administered by oral route.

Typical total daily doses of xipamide, or a pharmaceutically acceptable salt thereof are in the range of from 1 to 200 mg/day (expressed as xipamide free base), preferably from 10 to 80 mg/day. The compound may be administered by oral route.

Typical total daily doses of fabomotizole, or a pharmaceutically acceptable salt thereof are in the range of from 1 to 100 mg/day (expressed as fabomotizole free base), preferably from 10 to 60 mg/day. The compound may be administered by oral route.

Typical total daily doses of altizide, or a pharmaceutically acceptable salt thereof are in the range of from 1 to 1000 mg/day (expressed as altizide free base), preferably from 10 to 100 mg/day. The compound may be administered by oral route.

The compounds for use according to the invention may be administered as the sole active ingredient; or in combination with other active ingredients as is described elsewhere herein. In a particular embodiment, the compounds are used as the sole active ingredient. In another particular embodiment, the compounds are used in combination with other active ingredients as is described elsewhere herein.

The term "combination" refers to a product comprising one or more of the defined compounds, either in a single composition or in several compositions (or units), in which case the corresponding compounds are distributed among the several compositions.

When the combination is in the form of a single composition, the compounds present in the combination are always administered simultaneously.

When the combination is in the form of several compositions (or units), each of them having at least one but not all of the compounds of the combination, the compositions or (units) may be administered simultaneously, sequentially or separately.

Simultaneous administration means that the compounds or compositions (or units) are administered at the same time.

Sequential administration means that the compounds or compositions (or units) are administered at different time points in a chronologically staggered manner.

Separate administration means that the compounds or compositions (or units) are administered at different time points independently of each other.

The following examples represent specific embodiments of the present invention. They do not intend to limit in any way the scope of the invention defined in the present description.

### Examples

### Example 1 Utrophin upregulation in mdx^{5cv} myotubes

### Materials and methods

### Preparation of mdx myotubes

Myotube cultures were prepared from *mdx^{5cv}* mice, a mouse model of DMD generated by chemical mutagenesis that does not express dystrophin and has a more severe phenotype than the classical mdx mouse model (Jackson Laboratory; Beastrom et al. Am J Pathol 2011, 179:2464-2474; DOI: 10.1016/j.ajpath.2011.07.009). Isolation of satellite cell-derived primary myoblasts was performed via cell-surface antibodies and magnetic separation as previously described, and cultured for their expansion (Motohashi et al., J Vis Exp. 2014 Apr 8;(86):50846). Briefly, after collagenase (Worthington, CLS-2) treatment, dissociated cells from *mdx^{5cv}* mouse hindlimb muscle were incubated with anti-CD31 monoclonal antibody conjugated to phycoerythrin (eBioscience, 12-0311), anti-CD45 monoclonal antibody conjugated to phycoerythrin (eBioscience, 30-F11), anti-Sca1 monoclonal antibody conjugated to phycoerythrin (eBioscience, Dec-81) and anti-Integrin α7 (MBL International, ABIN487462), followed by anti-PE microbeads (Miltenyi Biotec, 130-048-801), and then performed LD column (Miltenyi Biotec, 130-042-901) separation. Negative cell populations were incubated with anti- Mouse IgG beads (Miltenyi Biotec, 130-048-402), and then MS column (Miltenyi Biotec, 130-042-201) separation was performed to isolate Integrin α7(+) satellite cells. Satellite cell-derived *mdx^{5cv}* myoblasts were maintained in culture on collagen coated plates in myoblast medium containing 20% FBS and 20 ng/ml bFGF (Invitrogen, PHG0263) in HAM's-F10 medium. Cell cultures were maintained in a humidified incubator at 37°C with 5% CO₂ and 5% O₂. The *mdx^{5cv}* myoblasts were induced to differentiate into multinucleated myotubes after low serum conditions (DMEM supplemented with 5% horse serum).

### Test compounds

Halofuginone was obtained from Selleck. Veliflapon and cloricromen HCl were obtained from Sigma. Fabomotizole HCl was obtained from MedChemExpress. Difenpiramide was obtained from Enamine. Amopyroquine and alpelisib were obtained from Carbosynth. Benorilate and xipamide were obtained from Fluorochem. Altizide was obtained from Toronto Research Chemicals. All chemical compounds were dissolved with 100% DMSO to adjust to 10 mM stock solutions.

### Treatment of myotubes

Every day compounds were added to *mdx^{5cv}* myotubes at different concentrations (0.01 to 10 µM) by replacement of the differentiation medium. After 3 days compound exposure, cells were harvested for RNA preparation by TRIzol reagent (15596026, ThermoFisher Scientific). Isolated RNA was used for cDNA synthesis using the Transcriptor First Strand cDNA synthesis kit (04379012001, Roche Molecular Diagnostics) using random primers. Diluted DMSO solution was used as a negative control vehicle. qRT-PCR assay for utrophin-A expression was performed using the following primers: Utro-F1 (Forward) 5'-CCTGATGATGGGCAGAACGAATTC-3' (SEQ ID NO: 1) and Utro-R1 (Reverse) 5'-CTGATGGGTGGTTTCCCACTCTTG-3' (SEQ ID NO: 2). 18S rRNA was used as an internal control, with the following primers: 18S-F1 (Forward) 5'-CGCACGGCCGGTACAGTGAAACTG-3' (SEQ ID NO: 3) and 18S-R1 (Reverse) 5'-CACCCGTGGTCACCATGGTAGGCA-3' (SEQ ID NO: 4). qPCR was performed using GoTaq qPCR Master Mix (A6001, Promega). All primers were synthesized as custom DNA oligos from Integrated DNA technologies (IDT). Analysis was done using the ΔCt method. Statistical analysis was performed with at least three independent experiments with between 2 and 4 replicates in each experiment.

### Results

The chemical compounds were tested in mdx^{5cv} myotubes at different concentrations for 3 days. Halofuginone was used as a positive control for the utrophin up-regulation. The molecules repeatedly showed significant up-regulation of utrophin expression (ranging from 5 to 44% depending on the compound and its concentration). Table 1 shows utrophin upregulation values in mdx^{5cv} myotubes by RT-PCR (values correspond to fold change utrophin vs DMSO expressed as average ± sem values).

**TABLE 1**

| Table 1: Utrophin upregulation in mdx^{5cv} myotubes treated with 0.1, 1 and 10 µM of different compounds (average of fold change utrophin vs DMSO ± sem values) | | | |
|---|---|---|---|
| **Treatment** | **0.1 µM** | **1 µM** | **10 µM** |
| Halofuginone (control) | 1.44 ± 0.37 | 1.24 ± 0.10 | 1.30 ± 0.04 |
| Veliflapon | 1.43 ± 0.24 | 1.35 ± 0.02 | 1.05 ± 0.15 |
| Difenpiramide | 1.24 ± 0.13 | 1.10 ± 0.21 | 1.23 ± 0.21 |
| Amopyroquine | 1.23 ± 0.11 | 1.10 ± 0.14 | 1.23 ± 0.37 |
| Cloricromen hydrochloride | 1.06 ± 0.19 | 1.07 ± 0.22 | 1.34 ± 0.13 |
| Alpelisib | 1.08 ± 0.09 | 1.45 ± 0.11 | 1.40 ± 0.13 |
| Benorilate | 1.16 ± 0.12 | 1.04 ± 0.16 | 1.58 ± 0.29 |
| Xipamide | 1.25 ± 0.18 | 1.05 ± 0.17 | 1.18 ± 0.10 |
| Fabomotizole hydrochloride | 1.34 ± 0.07 | 1.04 ± 0.10 | 1.34 ± 0.19 |
| Altizide | 1.09 ± 0.07 | 1.33 ± 0.09 | 1.33 ± 0.09 |

### Studies in human 3D skeletal muscle cultures

### Materials and methods

### Fabrication of human 3D skeletal muscle tissues and treatment

Human 3D skeletal muscle tissues were fabricated using polydimethylsiloxane (PDMS; Sylgard 184 silicone elastomer kit, Dow Corning) casting molds developed and fabricated as described in Fernández-Costa et al. 2022. Briefly, the casting molds design consists of a square-shaped pool, where the cells/hydrogel solution is seeded, and two vertical, flexible PDMS pillars. PDMS casting molds were prepared for muscle cell culture by first cleaning them by sonication on water with soap, 2-propanol, and Mili-Q water and sterilized with UV light for 15 min. It was followed by 1 h treatment with 1% Plurionic^{®} F-127 (Sigma-Aldrich) solution in cold PBS (100 µl/well) at 4 °C.

Human 3D skeletal muscle tissues were generated using Immortalized human muscle satellite cells (iHuMSCs) from a DMD patient (DMD 2, clone G82) (Massenet et al. 2020). iHuMSCs were cultured in growth medium (skeletal muscle basal medium (PromoCell GmbH), skeletal muscle supplemental mix (PromoCell GmbH), 10% Fetal Bovine Serum (FBS) (Gibco^{™}), 100 U/mL penicillin and 100 µg/ml streptomycin (Gibco^{™})). iHuMSCs were released from culture flasks via mild trypsinization. Cells were encapsulated at 2.5 ×107 cells/ml in a cell/hydrogel mixture of 30% v/v Corning^{®} Matrigel^{®} Growth Factor Reduced (GFR) Basement Membrane Matrix (Corning^{®}), 2 units of thrombin from human plasma (Sigma-Aldrich) and 4 mg/mL of fibrinogen from human plasma (50% v/v) (Sigma-Aldrich). 35 µl was pipetted within each PDMS casting mold using cool tips to avoid premature polymerization.

The cell/hydrogel mixture was polymerized for 30 min at 37°C followed by incubation in growth medium containing 1 mg/mL of 6-aminocaproic acid (ACA) (Sigma-Aldrich). ACA works as an antifibrinolytic, which avoids fibrin depolymerization. They were kept in growth media for two days and then switched to differentiation medium (Dulbecco's Modified Eagle Medium, high glucose, GlutaMAX^{™} Supplement (Gibco^{™}), containing 1% KnockOut^{™} Serum replacement (Gibco^{™}), 1% Insulin-Transferrin-Selenium-Ethanolamine (Gibco^{™}) and 1% Penicillin-Streptomycin-Glutamine (Gibco^{™})) containing 1 mg/mL of ACA for seven days. Half of the volume of differentiation medium was replaced every two days. After five days of differentiation, the medium was replaced by differentiation medium with the drugs at 10 µM and 0.01% DMSO final concentration. The treatment was kept for 48 hours.

### Example 2 Increase of the force of muscle contraction in response to electrical stimulation

### Electrical Pulse Stimulation (EPS) and video analysis

Human 3D skeletal muscle tissues were electrically stimulated after seven days of differentiation to induce muscle contraction. A custom-made device consisted of a 12-well plate with two graphite electrodes per well located on the lid was used to perform the EPS. PDMS casting molds were placed on a 12-well plate with fresh differentiation medium. The plate was placed on a Zeiss Axio Observer Z1/7 outfitted with the XL S1 cell incubator at 37 °C and 5% CO2. The device was connected to a pulse generator (Multifunction Generator WF1974, NF Corporation), and tissues were stimulated with electrical square-wave pulses of 1 V/mm, 1 ms of pulse width, and frequencies varying from 1 to 50 Hz. The contractibility in response of EPS of 1 Hz of frequency was evaluated to study twitch contraction dynamics. On the other hand, 50 Hz of frequency was applied to induce tetanic contractions.

Videos of the movement of the pillar of the casting molds in response to the muscle contractions were taken with a ZEISS Axio Observer Z1/7 microscope. Cross-sectional area and location of the tissues on the axis of the pillars were also recorded during the assay. Videos were analyzed using a custom-made Python script and the specific force performed from the tissues was calculated applying Euler's displacement theorem together with the calculated displacement of the pillar as done in Mestre et al. 2018. Tetanic index was calculated as the ratio between the maximal tetanic specific force divided by the maximal twitch specific force.

Bioengineered DMD patient-derived 3D skeletal muscle tissues were differentiated and treated with 10 µM of veliflapon or difenpiramide for 2 days prior to determination of the force of muscle contraction in response to electrical stimulation. Muscle tissues treated with 0.01% DMSO were used as negative control.

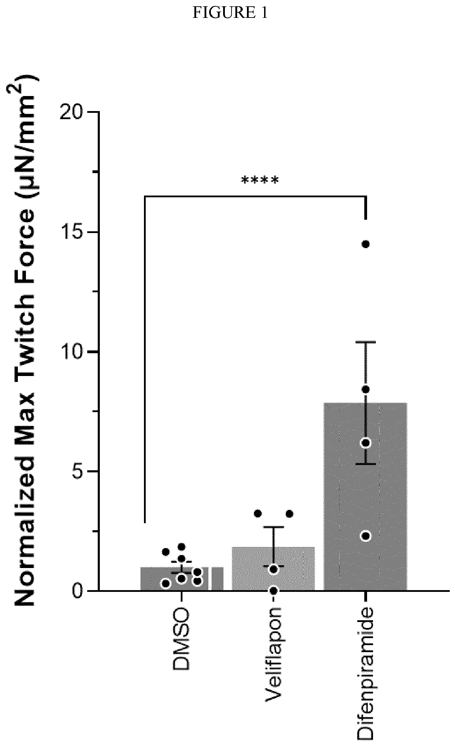

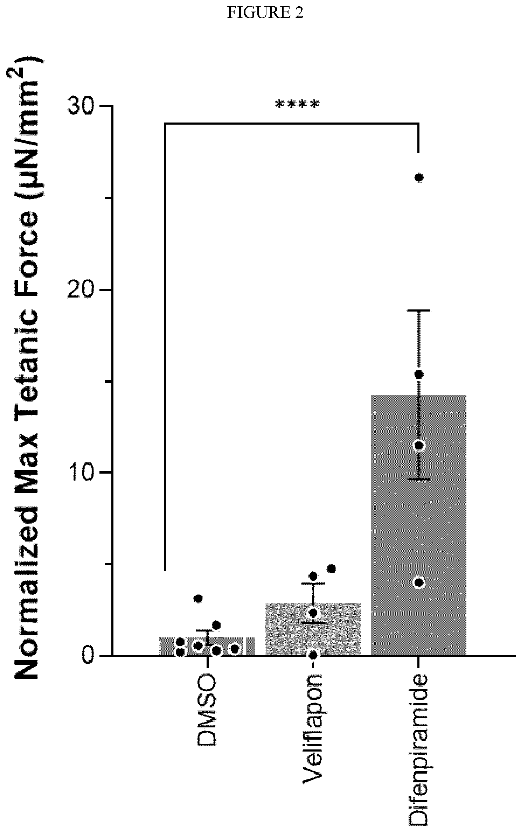

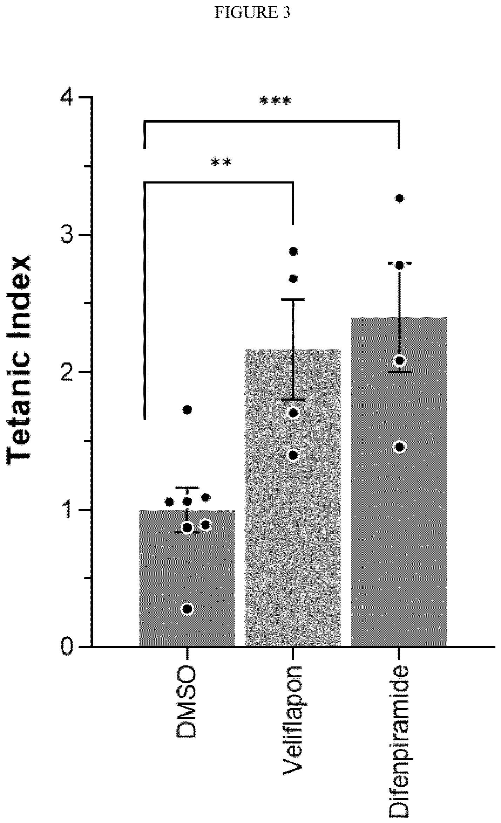

The results are shown in Figures 1 to 3 as follows: Improvement of muscle function in DMD patient-derived 3D skeletal muscle tissues: Immortalized DMD patient-derived 3D skeletal muscle tissues were differentiated for 5 days and treated with 10 µM veliflapon, 10 µM difenpiramide or 0.01% DMSO for 2 days. Muscles were electrically stimulated and the maximal twitch (Figure 1) or tetanic (Figure 2) contractile force in response to electrical stimulation was measured and compared with the force obtained in DMSO treated muscles. Graphs represent maximal contractile force normalized by area of tissue as average values ± sem. Tetanic index (Figure 3) was calculated and represented as average values ± sem. Statistical significance calculated by Anova and corrected by Dunnett's test is represented by P value: ** (P ≤ 0.01);*** (P ≤ 0.001; **** (P ≤ 0.0001).

Treatment with veliflapon and difenpiramide improved muscle function, as shown by an increase of the force of muscle contraction in response to electrical stimulation as shown in Figures 1 to 3. An increase in both twitch (Fig 1) and tetanic (Fig 2) contractile force was observed after treatment with veliflapon and difenpiramide compared with DMSO treated cells. Also, a significant increase in the tetanic index (Fig 3) was observed after treatment with the two compounds.

### Example 3. Utrophin expression using immunohistochemistry Cryosectioning

Human 3D skeletal muscle tissues were fixed in 10% formalin solution (approx. 4% formaldehyde) (Sigma-Aldrich) for 30 min at RT and washed three times with PBS for 5 min. Samples were removed from the PDMS platform and placed on 30% sucrose solution in PBS for 48h at 4 °C in agitation. They were frozen by embedding them in optimal cutting temperature compound (OCT compound) (PolyFreeze, Sigma-Aldrich) within a disposable plastic Cryomold^{®} (VWR) using a batch of isopentane chilled by liquid nitrogen. Samples were stored at -20 °C, until sectioning. They were sectioned with a cryostat (Leica CM1900) to obtain transverse sections at a thickness of 20 µm that were placed on SuperFrost Plus^{™} Adhesion slides (Fisher Scientific) and stored at -20 °C until staining was performed.

### Immunohistochemistry

Slides containing sections of tissue were encircled with a PAP pen (ImmEdge^{™}, Vector laboratories) and they were permeabilized with PBS-T (0.1% Triton-X (Sigma-Aldrich) in PBS) for 10min, followed by incubation with blocking buffer (0.3% Triton-X, 3% donkey serum (Sigma-Aldrich) in PBS) for 30 min at RT. Next, samples were incubated with the primary antibody (Monoclonal mouse Anti-utrophin primary antibody (MANCHO7) in blocking buffer at 4 °C overnight. After 3 PBS-T washes of 5 min, the samples were incubated for 45 min at RT with Polyclonal donkey anti-mouse IgG, Alexa Fluor^{™} 488-conjugated secondary antibody. Tissue sections were mounted with Fluoromount-G^{™} Mounting Medium with DAPI (Thermo Fisher). Nail polish was used to seal the edges of coverslips.

### Imaging and image analysis

Fluorescence images were taken with a ZEISS LSM800 confocal laser scanning microscope. Images were analyzed using the Fiji image processing package, a distribution of Image J (Schindelin et al., 2012). For fluorescence quantification, mean grey values of each image was measured (minimum of 4 images per tissue).

### Statistics

Results are presented as mean ± sem. Statistical significance was determined using GraphPad Prism by one-way ANOVA and Dunnett's multiple comparison test. p < 0.05 was considered statistically significant.

### Results

Bioengineered DMD patient-derived 3D skeletal muscle tissues were differentiated and treated with 10 µM of veliflapon or difenpiramide for 2 days prior to determination of utrophin expression. Muscle tissues treated with 0.01% DMSO were used as negative control.

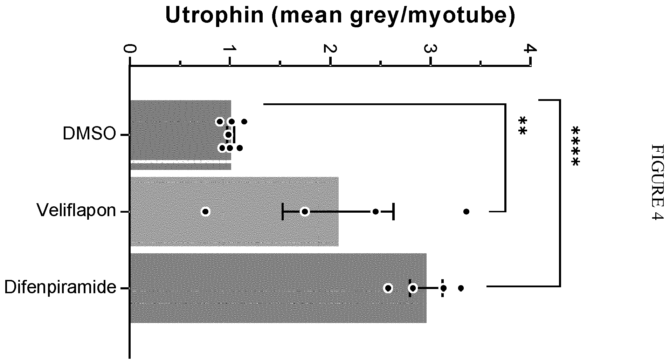

The results are shown in Figure 4 as follows: Increase of utrophin expression in human DMD cultures: Immortalized DMD patient-derived 3D skeletal muscle tissues were differentiated for 5 days and treated with 10 µM veliflapon, 10 µM difenpiramide or 0.01% DMSO for 2 days. Utrophin expression in muscle slices was determined by immunohistochemistry and compared with the signal observed in DMSO treated cells. Graphs represent fold increase of fluorescence values vs DMSO expressed as average ± sem. Statistical significance calculated by Anova and corrected by Dunnett's test is represented by P value: ** (P ≤ 0.01); **** (P ≤ 0.0001).

Utrophin expression increased up to 3 fold after treatment of human DMD 3D muscle tissues with veliflapon and difenpiramide compared with DMSO treated cells, confirming previous results in mdx myotubes (Table 1).

Overall, these results indicate that the compounds are useful for increasing the expression of utrophin and improving muscle function, indicating their potential for the treatment of dystrophinopathies.

## Claims

1. Compound selected from the group consisting of Difenpiramide, Veliflapon, Amopyroquine, Cloricromen, Alpelisib, Benorilate, Xipamide, Fabomotizole and Altizide, or a pharmaceutically acceptable salt thereof, for use in the treatment or prevention of a dystrophinopathy.

2. Compound for use according to claim 1, wherein the compound is selected from the group consisting of Difenpiramide, Veliflapon, Cloricromen, Alpelisib, Fabomotizole, or a pharmaceutically acceptable salt thereof.

3. Compound for use according to any one of claims 1 and 2, wherein the compound is Difenpiramide or a pharmaceutically acceptable salt thereof.

4. Compound for use according to any one of claims 1 and 2, wherein the compound is Veliflapon or a pharmaceutically acceptable salt thereof.

5. Compound for use according to any one of claims 1 and 2, wherein the compound is Cloricromen or a pharmaceutically acceptable salt thereof.

6. Compound for use according to any one of claims 1 and 2, wherein the compound is Alpelisib or a pharmaceutically acceptable salt thereof.

7. Compound for use according to any one of claims 1 and 2, wherein the compound is Fabomotizole or a pharmaceutically acceptable salt thereof.

8. Combination comprising two or more agents selected from the group consisting of Difenpiramide, Veliflapon, Amopyroquine, Cloricromen, Alpelisib, Benorilate, Xipamide, Fabomotizole, Altizide, or a pharmaceutically acceptable salt thereof; ataluren, deflazacort, givinostat, pizuglanstat, ifetroban, vamorolone, idebenone, prednisone, prednisolone, 20-hydroxyecdysone, metformin and L-citrulline combination, MA-0211, tamoxifen, dextro epicatechin, exaluren, ARM-210, 2,6-disubstituted-pyiridazin-3(2H)-ones, N-Benzyl-p-toluenesulphonamide, MP-101, MT-002, rimeporide, tetracosactide hexaacetate, elamipretide, KER-050, SRP-5051, TXA-127, PB-1023, pamrevlumab, eteplirsen, golodirsen, vitolarsen, casimersen, ATL-1102, NS-089, renadirsen, WVEN-531, fordadistrogene movaparvovec, GNT-004, AAV1-follistatin viral vector, delandistrogene moxeparvovec, AT-702, AAVrh74-GALGT2, SGT-001, CAP-2003, CAP-1002; wherein at least one of said agents is selected from the group consisting of Veliflapon, Difenpiramide, Amopyroquine, Cloricromen, Alpelisib, Benorilate, Xipamide, Fabomotizole and Altizide, or a pharmaceutically acceptable salt thereof for use in the treatment or prevention of a dystrophinopathy.

9. Combination for use according to claim 8, wherein at least one of said agents is selected from the group consisting of Difenpiramide, Veliflapon, Cloricromen, Alpelisib, Fabomotizole, or a pharmaceutically acceptable salt thereof.

10. Pharmaceutical composition comprising:
- a compound selected from the group consisting of Difenpiramide, Veliflapon, Amopyroquine, Cloricromen, Alpelisib, Benorilate, Xipamide, Fabomotizole and Altizide, or a pharmaceutically acceptable salt thereof; or a combination as defined in any one of claims 8 to 9; and
- at least one pharmaceutically acceptable excipient,
for use in the treatment or prevention of a dystrophinopathy.

11. A pharmaceutical composition for use according to claim 10, wherein the pharmaceutical composition is administered by oral or parenteral route.

12. Compound for use according to any one of claims 1 to 7, or combination for use according to any one of claims 8 to 9, or pharmaceutical composition for use according to any one of claims 10 to 11, wherein the dystrophinopathy is selected from Duchenne muscular dystrophy, Becker muscular dystrophy, Duchenne muscular dystrophy-associated dilated cardiomyopathy or cramps with myoglobinuria.

13. Compound for use according to any one of claims 1 to 7, or combination for use according to any one of claims 8 to 9, or pharmaceutical composition for use according to any one of claims 10 to 11, wherein the dystrophinopathy is Duchenne muscular dystrophy.

14. Compound for use according to any one of claims 1 to 7 and 12, 13; or combination for use according to any one of claims 8, 9, 12 and 13; or pharmaceutical composition for use according to any one of claims 10 to 13; wherein the treatment or prevention of the dystrophinopathy is in a pediatric human subject.

## Patentansprüche

1. Verbindung, ausgewählt aus der Gruppe bestehend aus Difenpiramid, Veliflapon, Amopyroquin, Cloricromen, Alpelisib, Benorilat, Xipamid, Fabomotizol und Altizid, oder einem pharmazeutisch akzeptablen Salz davon zur Verwendung bei der Behandlung oder Vorbeugung einer Dystrophinopathie.

2. Verbindung zur Verwendung nach Anspruch 1, wobei die Verbindung aus der Gruppe bestehend aus Difenpiramid, Veliflapon, Cloricromen, Alpelisib, Fabomotizol, oder einem pharmazeutisch akzeptablen Salz davon ausgewählt ist.

3. Verbindung zur Verwendung nach einem der Ansprüche 1 und 2, wobei die Verbindung Difenpiramid oder ein pharmazeutisch akzeptables Salz davon ist.

4. Verbindung zur Verwendung nach einem der Ansprüche 1 und 2, wobei die Verbindung Veliflapon oder ein pharmazeutisch akzeptables Salz davon ist.

5. Verbindung zur Verwendung nach einem der Ansprüche 1 und 2, wobei die Verbindung Cloricromen oder ein pharmazeutisch akzeptables Salz davon ist.

6. Verbindung zur Verwendung nach einem der Ansprüche 1 und 2, wobei die Verbindung Alpelisib oder ein pharmazeutisch akzeptables Salz davon ist.

7. Verbindung zur Verwendung nach einem der Ansprüche 1 und 2, wobei die Verbindung Fabomotizol oder ein pharmazeutisch akzeptables Salz davon ist.

8. Kombination, die zwei oder mehr Wirkstoffe umfasst, ausgewählt aus der Gruppe bestehend aus Difenpiramid, Veliflapon, Amopyroquin, Cloricromen, Alpelisib, Benorilat, Xipamid, Fabomotizol, Altizid oder einem pharmazeutisch akzeptablen Salz davon; Ataluren, Deflazacort, Givinostat, Pizuglanstat, Ifetroban, Vamorolon, Idebenon, Prednison, Prednisolon, 20-Hydroxyecdyson, Metformin und L-Citrullin Kombination, MA-0211, Tamoxifen, Dextro-Epicatechin, Exaluren, ARM-210, 2,6-disubstituiertes Pyridazin-3(2H)-on, N-Benzyl-p-toluolsulfonamid, MP-101, MT-002, Rimeporid, Tetracosactid-Hexaacetat, Elamipretid, KER-050, SRP-5051, TXA-127, PB-1023, Pamrevlumab, Eteplirsen, Golodirsen, Vitolarsen, Casimersen, ATL-1102, NS-089, Renadirsen, WVEN-531, Fordadistrogen Movaparvovec, GNT-004, AAV1-Follistatin viraler Vektor, Delandistrogen moxeparvovec, AT-702, AAVrh74-GALGT2, SGT-001, CAP-2003, CAP-1002; wobei mindestens einer der genannten Wirkstoffe aus der Gruppe bestehend aus Veliflapon, Difenpiramid, Amopyroquin, Cloricromen, Alpelisib, Benorilat, Xipamid, Fabomotizol und Altizid, oder einem pharmazeutisch akzeptablen Salz davon ausgewählt ist zur Verwendung bei der Behandlung oder Vorbeugung einer Dystrophinopathie.

9. Kombination zur Verwendung nach Anspruch 8, wobei mindestens einer dieser Wirkstoffe aus der Gruppe bestehend aus Difenpiramid, Veliflapon, Cloricromen, Alpelisib, Fabomotizol oder einem pharmazeutisch akzeptablen Salz davon ausgewählt ist.

10. Pharmazeutische Zusammensetzung, umfassend
- eine Verbindung, ausgewählt aus der Gruppe bestehend aus Difenpiramid, Veliflapon, Amopyroquin, Cloricromen, Alpelisib, Benorilat, Xipamid, Fabomotizol und Altizid, oder einem pharmazeutisch akzeptablen Salz davon, oder eine Kombination wie in einem der Ansprüche 8 bis 9 definiert, und
- mindestens einen pharmazeutisch akzeptabler Hilfsstoff,
zur Behandlung oder Vorbeugung einer Dystrophinopathie.

11. Pharmazeutische Zusammensetzung zur Verwendung nach Anspruch 10, wobei die pharmazeutische Zusammensetzung oral oder parenteral verabreicht wird.

12. Verbindung zur Verwendung nach einem der Ansprüche 1 bis 7, oder Kombination zur Verwendung nach einem der Ansprüche 8 bis 9, oder pharmazeutische Zusammensetzung zur Verwendung nach einem der Ansprüche 10 bis 11, wobei die Dystrophinopathie ausgewählt ist aus Duchenne-Muskeldystrophie, Becker-Muskeldystrophie, Duchenne-Muskeldystrophie-assoziierter dilatativer Kardiomyopathie oder Krämpfen mit Myoglobinurie.

13. Verbindung zur Verwendung nach einem der Ansprüche 1 bis 7, oder Kombination zur Verwendung nach einem der Ansprüche 8 bis 9, oder pharmazeutische Zusammensetzung zur Verwendung nach einem der Ansprüche 10 bis 11, wobei die Dystrophinopathie Duchenne-Muskeldystrophie ist.

14. Verbindung zur Verwendung nach einem der Ansprüche 1 bis 7 und 12, 13, oder Kombination zur Verwendung nach einem der Ansprüche 8, 9, 12 und 13, oder pharmazeutische Zusammensetzung zur Verwendung nach einem der Ansprüche 10 bis 13, wobei die Behandlung oder Vorbeugung der Dystrophinopathie bei einem pädiatrischen Menschen erfolgt.

## Revendications

1. Composé choisi parmi le groupe constitué de difenpiramide, véliflapon, amopyroquine, cloricromène, alpélisib, bénorilate, xipamide, fabomotizole et altizide, ou un sel de ceux-ci acceptable du point de vue pharmaceutique, pour une utilisation dans le traitement ou la prévention d'une dystrophinopathie.

2. Composé pour une utilisation selon la revendication 1, dans lequel le composé est choisi parmi le groupe constitué de difenpiramide, véliflapon, cloricromène, alpélisib, fabomotizole ou un sel de ceux-ci acceptable du point de vue pharmaceutique.

3. Composé pour une utilisation selon l'une quelconque des revendications 1 et 2, dans lequel le composé est le difenpiramide ou un sel de celui-ci acceptable du point de vue pharmaceutique.

4. Composé pour une utilisation selon l'une quelconque des revendications 1 et 2, dans lequel le composé est le véliflapon ou un sel de celui-ci acceptable du point de vue pharmaceutique.

5. Composé pour une utilisation selon l'une quelconque des revendications 1 et 2, dans lequel le composé est le cloricromène ou un sel de celui-ci acceptable du point de vue pharmaceutique.

6. Composé pour une utilisation selon l'une quelconque des revendications 1 et 2, dans lequel le composé est l'alpélisib ou un sel de celui-ci acceptable du point de vue pharmaceutique.

7. Composé pour une utilisation selon l'une quelconque des revendications 1 et 2, dans lequel le composé est le fabomotizole ou un sel de celui-ci acceptable du point de vue pharmaceutique.

8. Combinaison comportant deux agents ou plus choisis parmi le groupe constitué de difenpiramide, véliflapon, amopyroquine, cloricromène, alpélisib, bénorilate, xipamide, fabomotizole et altizide, ou un sel de ceux-ci acceptable du point de vue pharmaceutique ; ataluren, déflazacort, givinostat, pizuglanstat, ifétroban, vamorolone, idébénone, prednisone, prednisolone, 20-hydroxyecdysone, combinaison metformine et L-citrulline, MA-0211, tamoxifène, dextro épicatéchine, exalurène, ARM-210, 2,6-disubstitué-pyiridazin-3(2H)-ones, N-benzyl-p-toluènesulfonamide, MP-101, MT-002, riméporide, tétracosactide hexa-acétate, élamiprétide, KER-050, SRP-5051, TXA-127, PB-1023, pamrevlumab, eteplirsen, golodirsen, vitolarsen, casimersen, ATL-1102, NS-089, renadirsen, WVEN-531, fordadistrogène movaparvovec, GNT-004, vecteur viral AAV1-follistatine, delandistrogène moxeparvovec, AT-702, AAVrh74-GALGT2, SGT-001, CAP-2003, CAP-1002; dans laquelle au moins un desdits agents est choisi parmi le groupe constitué de véliflapon, difenpiramide, amopyroquine, cloricromène, alpélisib, bénorilate, xipamide, fabomotizole et altizide, ou un sel de ceux-ci acceptable du point de vue pharmaceutique, pour une utilisation dans le traitement ou la prévention d'une dystrophinopathie.

9. Combinaison pour une utilisation selon la revendication 8, dans laquelle au moins un desdits agents est choisi parmi le groupe constitué de difenpiramide, véliflapon, cloricromène, alpélisib, fabomotizole ou un sel de ceux-ci acceptable du point de vue pharmaceutique.

10. Composition pharmaceutique comportant :
- un composé choisi parmi le groupe constitué de difenpiramide, véliflapon, amopyroquine, cloricromène, alpélisib, bénorilate, xipamide, fabomotizole et altizide, ou un sel de ceux-ci acceptable du point de vue pharmaceutique, ou une combinaison selon l'une quelconque des revendications 8 à 9, et
- au moins un excipient acceptable du point de vue pharmaceutique,
pour une utilisation dans le traitement ou la prévention d'une dystrophinopathie.

11. Composition pharmaceutique pour une utilisation selon la revendication 10, dans laquelle la composition pharmaceutique est administrée par voie orale ou parentérale.

12. Composé pour une utilisation selon l'une quelconque des revendications 1 à 7, ou combinaison pour une utilisation selon l'une quelconque des revendications 8 à 9, ou composition pharmaceutique pour une utilisation selon l'une quelconque des revendications 10 à 11, dans lesquels la dystrophinopathie est choisie parmi la dystrophie musculaire de Duchenne, la dystrophie musculaire de Becker, la cardiomyopathie dilatée associée à la dystrophie musculaire de Duchenne ou des crampes avec myoglobinurie.

13. Composé pour une utilisation selon l'une quelconque des revendications 1 à 7, ou combinaison pour une utilisation selon l'une quelconque des revendications 8 à 9, ou composition pharmaceutique pour une utilisation selon l'une quelconque des revendications 10 à **11,** dans lesquels la dystrophinopathie est la dystrophie musculaire de Duchenne.

14. Composé pour une utilisation selon l'une quelconque des revendications 1 à 7 et 12, 13, ou combinaison pour une utilisation selon l'une quelconque des revendications 8, 9, 12 et 13, ou composition pharmaceutique pour une utilisation selon l'une quelconque des revendications 10 à 13, dans lequel le traitement ou la prévention de la dystrophinopathie est chez un sujet pédiatrique humain.
